# EUROPEAN PATENT SPECIFICATION

(11) **EP 0 972 031 B1**
(45) Date of publication and mention of the grant of the patent: **27.04.2011**
(21) Application number: 98906998.4
(22) Date of filing: 02.03.1998
(51) Int. Cl.: C12N 15/12, C07K 14/78, C12N 15/62, C12N 5/10, A61K 48/00

(54) **PROCOLLAGEN ASSEMBLY**
ZUSAMMENFUEGUNG VON PROKOLLAGEN
ASSOCIATION PROCOLLAGENE

(30) Priority: 01.03.1997 GB 9704305
(43) Date of publication of application: 19.01.2000
(73) Proprietor: The University of Manchester, Manchester M13 9PL (GB)
(72) Inventor: BULLEID, Neil, John, Manchester M20 6SY (GB)
(74) Representative: Atkinson, Peter Birch
(86) International application number: PCT/GB1998/000468
(87) International publication number: WO 1998/038303

(56) References cited:
- EP-A- 0 699 752
- WO-A-93/07889
- WO-A-97/08311
- LEES J F AND BULLEID N J: "The role of cysteine residues in the folding and association of the COOH-terminal propeptide of types I and III procollagen" JOURNAL OF BIOLOGICAL CHEMISTRY, vol. 269, no. 39, 30 September 1994, pages 24354-24360, XP002071356 cited in the application
- LEES J F ET AL: "Identification of the molecular recognition sequence which determines the type-specific assembly of procollagen" EMBO JOURNAL, vol. 16, no. 5, 3 March 1997, pages 908-916, XP002071357
- MYLLYHARJU J ET AL: "Expression of wild-type and modified proalpha chains of human type I collagen in insect cells leads to the formation of ..." JOURNAL OF BIOLOGICAL CHEMISTRY, vol. 272, no. 35, 29 August 1997, pages 21824-21830, XP002071358

## Description

The present invention relates to a method of regulating assembly of procollagens.

Most cells, whether simple unicellular organisms or cells from human tissue, are surrounded by an intricate network of macromolecules which is known as the extracellular matrix and which is comprised of a variety of proteins and polysaccharides. The major protein component of this matrix is a family of related proteins called the collagens which are thought to constitute approximately 25% of total proteins in mammals. There are at least 20 genetically distinct types of collagen molecule, some of which are known as fibrillar collagens (collagen types I, II, III, V and XI) because they typically form large fibres, known as collagen fibrils, that may be many mircometers long and may be visualised by electron microscopy.

Collagen fibrils arc comprised of polymers of collagen molecules and are produced by a process which involves conversion of procollagen to collagen molecules which then assemble to form the polymer. Procollagen consists of a triple stranded helical domain in the centre of the molecule and has non-helical region at the amino terminal (known as the N-terminal propeptide) and at the carboxy terminal (known as the C-terminal propeptide). The triple stranded helical domain is made up of three polypeptides which are known as α chains. Procollagen is synthesised intracellularly from pro-α chains (α chains with N- and C-terminal propeptide domains) on membrane-bound ribosomes following which the pro-α chains are inserted into the endoplasmic reticulum.

Within the endoplasmic reticulum the pro-α chains are assembled into procollagen molecules. This assembly can be divided into two stages an initial recognition, event between the pro-α chains which determines chain selectively and then a registration event which leads to correct alignment of the triple helix. Procollagen assembly is initiated by association of the C-terminal propeptide domains of each pro-α chain to form the C- terminal propeptide. Assembly of the triple helix domain then proceeds in a C- to N- terminal direction and is compteted by formation of the N- terminal propeptide. The mature procollagen molecules are ultimately secreted into the extracellular environment where they are converted into collagen by the action of Procollagen N-Proteinases (which cleave the N-terminal propeptide) and Procollagen C-Proteinascs (which cleave the C-terminal propeptide). Once the propeptides have been removed the collagen molecules thus formed are able to aggregate spontaneously to form the collagen fibrils.

Collagens have many uses industrially. For instance, Collagen gels can be formed from collagen fibrils *in vitro* and may be used to support cell attachment. Such gels may be used in cell culture to maintain the phenotype of certain cells, such as chondrocytes explanted from cartilage. Collagen may be also used as a "stuffer" or packing agent surgically and is particularly known to be used in cosmetic surgery, for enlarging the appearance of lips for instance. *In vivo,* collagen is a major component of the extracellular matrix and serves a multitude of purposes. Numerous diseases are known which involve abnormalities in collagen synthesis and regulation. Procollagens may be used (or be of potential use) for the treatment of these diseases.

Large quantifies of procollagens need to be synthesised to meet increasing industrial demand. A convenient means of synthesizing procollagens is by expression of exogenous pro-α chains in a host cell followed by the assembly of pro-α chains into the procollagen. For this to occur it is necessary to ensure that any host cell used has the necessary post-transtational facilities required to assemble procollagens from pro-α chains. This may be achieved by expression in cells which normally synthesise procollagen. However one problem in such systems is that endogenously expressed pro-α chains can co-assemble with the exogenously introduced pro-α chains giving rise to undesirable hybrid molecules.

In other circumstances it may be desirable to generate two or more procollagens from distinct pro-α chains of an exogenous source in a host cell in which case it is required that co-assembly of pro-α chains to form undesirable hybrid molecules should not occur.

It is also conceivable that procollagens may need to be assembled in a cell-free system *in vitro,* in which case co-assembly of pro-α chains giving rise to undesirable hybrid molecules also needs to be avoided.

It is an object of the present invention to provide a means by which pro-α chains may be assembled into desired procollagens without undesirable co-assembling with other pro-α chains.

According to the present invention there is provided a method of producing a first procollagen comprising expressing, in a system which co-expresses and assembles at least one second procollagen, exogenously selected or exogenously manipulated genes which express pro-α chains with domains which have the activity of C-terminal propeptide domains for assembly into the first procollagen but which will not co-assemble with the C- terminal propeptide of the pro-α chains that assemble to form the said at least one second procollagen, said genes including one which has been exogenously manipulated so as to express a pro-α chain which comprises
(i) a first moiety having the activity of a C-terminal propetide domain and incorporating a recognition sequence of a first type pro-α chain conferring a selectivity on the assembly of the pro-α chain into a procollagen; and
(ii) a second moiety containing a triple helix forming domain from a pro-α chain different from said first type,
said first moiety being attached to said second moiety so that said recognition sequence permits co-assembly of said pro-α chain for assembly into said first procollagen with other pro-α chains having said activity and a triple helix forming domain, whereby said first procollagen is produced, said method being other than a method of therapeutic treatment.

Thus the exogenously selected or exogenously manipulated genes may express pro-α chains that may be assembled into trimers to form procollagen molecules which in turn may be formed into collagen polymers following exposure to Procollagen C-Proteinase and Procollagen N-Protcinases (which respectively leave the C- and N- terminal propeptides from the procollagen molecules to form monomers which aggregate spontaneously to form the collagen polymers). The collagen polymer is preferably a fibrillar collagen.

The invention is based upon the recognition by the inventors that a crucial stage in the assembly of procollagens is an initial recognition step between pro-α chains which ensures that pro-α chains assemble in a type-specific manner. This recognition step involves a recognition sequence in the C- terminal propetide domain of pro-α chains. For instance, a single cell may synthesise several collagen types and, therefore, several different pro-α chains, yet these chains are able to discriminate between C- terminal propetide domains to ensure type-specific assembly. One example, of this discrimination can be found in cells expressing both type I and type III procollagen. Here at least three pro-α chains are synthesised, namely proα1(I), proα2(I) and proα1(III) chains. However the only procollagens formed are proα1(I)]₂proα2(I) heterotrimers and [proα1(III)]₃ homotrimers. Other combinations of pro-α chains do not assemble into procollagen.

In PCT/GB96/02122 (WO-A-97/08311) we have disclosed that specific regions within the C-terminal, propeptide are the recognition sequences involved in the specificity of association between C-terminal propeptide domains of pro-α chains during the formation of procollagens. These recognition sequences were identified as having the following amino acid sequences for each respective pro-α chain:
pro-α1(I) GGQGSDPAOV AIQLTFLRLM STE
pro-α2 (f) NVEGVTSKEM ATQLAFMRLL ANY
pro-α1 (II) GDDNLAPNTA NVQMTFLRLL STE
pro-α1 (III) GNPELPEDVL DVQLAFLRLL SSR
pro-α1 (V) VDAEGNPVGV .VQMTFLRLL SAS
pro-α2 (V) GDHQSPNTAI .TQMTFLRLL SKE
pro-α1 (XI) LDVEGNSINM .VQMTFLKLL TAS
pro-α2 (XI) VDSEGSPVGV .VQLTFLRL SVS

These recognition sequences confer selectivity and specificity of pro-α chain association.

In accordance with the invention, we have devised methods by which desired pro-α chains can be expressed and assembled into procollagens in a system which co-expresses and assembles pro-α chains or at least one further procollagen without undesired co-assembly producing unwanted hybrid molecules. This is effected by exogenously manipulating or selecting the gene or genes that encode for the desired pro-α chains such that the domains having C- terminal propeptide activity of these pro-α chains that are expressed from the manipulated or selected gene or genes will not associate with (and therefore not co-assemble with) the domains having C- terminal propeptide activity of the pro-α chains of the said at least one further procollagen. Put alternatively, the domains having C- terminal propeptide activity of the pro-α chain expressed by the manipulated or selected gene arc such that association between pro-α chains expressed from such a gene and association between at least one pro-α chain which forms the further procollagen is mutually exclusive.

Thus, in accordance with the present invention, a gene for expressing a pro-α chain for assembly into a desired procollagen may be exogenously selected or constructed to express a pro-α chain comprised of (i) a first moiety incorporating at least the recognition sequence of the C-terminal propeptide domain of a first type of pro-α chain, and (ii) a second moiety, attached to the first moiety which will assemble into the desired procollagen. The second moiety preferably is at least partially capable of trimerising to form a triple helix. More preferably the second comprises at least some amino acids capable of trimerising with other α chains. The expressed molecule is one which has been "engineered" (by appropriate selection of the first and second moieties) such that it may be expressed and assembled in a system which co-expresses and assembles at least one further type of pro-α chain without undesirable formation of hybrid molecules.

The domain having C-propeptide activity expressed by the exogenously selected or modified gene may comprise a recognition sequence as listed above. The domain may be a modification (e.g. by substitution or deletion) of such a recognition sequence, the domain retaining C-propeptide activity.

To prepare exogenously modified genes for use in the method of the invention, the DNA encoring for the desired recognition sequence may be substituted for the DNA encoding recognition sequences found in natural or artificially constructed pro-α chain genes to form an exogenously modified gene for use in the method of the invention.

DNA. particularly DNA, encoding natural pro-α chains is known and available in the art. For example, WO-A-9307889, WO-A-9416570 and the references cited in both of them give details. Such DNA may be used as a convenient starting point for making a DNA molecule that encodes for an exogenously manipulated gene for use in the invention.

DNA sequences, cDNAs, full genomic sequences and minigencs (genomic sequences containing some, but not all, of the introns present in the full length gene) may be inserted by recombinant means into a DNA sequence coding for naturally occurring pro-α chains (such as the starting point DNA mentioned above) to form the DNA molecule that encodes for an exogenously manipulated gene for use according to the first aspect of the invention. Because of the large number of introns present in collagen genes in general, experimental practicalities will usually favour the use of cDNAs or, in some circumstances, minigenes. The inserted DNA sequences, cDNAs, full genomic sequences or minigenes code for amino acids which give rise to pro-α chains with a C- terminal propeptide domain which will not co-assemble with the C- terminal propeptide domain of the pro-α chains that assemble to form the said at least one further procollagen.

Preferred exogenous manipulations of the gene or genes involve alteration of the recognition sequence within the C- terminal propeptidc domain which is responsible for selective association of pro-α chains such that any pro-α chain expressed from the manipulated gene will not undesirably co-assemble with pro-α chains endogenously expressed from a host cell into which the exogenously manipulated gene or genes is or are introduced.

In our previous application PCT/GB96/02122 (WO-A-97/083 11) we disclosed novel molecules comprising combinations of natural or novel C- terminal propeptide domains with alien α chains (or a non-collagen material). PCT/GB96/02122 also disclosed DNA molecules encoding such molecules. These DNA molecules may be used according to the methods of the current invention. Such molecules disclosed in PCT/GB96/02122 are incorporated herein by reference.

Alternatively deletion, addition or substitution mutations may be made within the DNA encoding for any one of these recognition sequences which altcr selectivity and specificity of pro-α chain association.

Other preferred exogenous manipulations of a gene involve the construction of gene constructs which encode for chimeric pro-α chains formed from the genetic code of at least two different pro-α chains. It is particularly preferred that the chimeric pro-α chains or derivatives thereof comprise a recognition sequence from the C- terminal propeptide domain of one type of pro-α chain and the α chain domain from another type of pro-α chain. Preferred chimeric pro-α chains comprise the recognition sequence of a pro-α1 (I), pro-α2 (I). pro-α1 (II), pro-α1 (III), pro-α1 (V), pro-α2 (V), pro-α1 (XI) or pro-α2 (XI) pro-α chain and an α-chain domain selected from a different one of these pro-α chains Most preferred pro-α chains for making chimeric pro-α chains are those which form collagens I and III particularly pro-α2 (1) and pro-α1 (III). Specific preferred chimeric pro-α chains are disclosed in the Example.

In a preferred exogenous manipulation of a gene according to the methods of the invention, the DNA encoding for the recognition sequence of the proα2(I) chain gene can be replaced with the corresponding DNA encoding for the recognition sequence of the proα1(III) chain gene and this manipulated gene can be expressed and assembled to form procollagens which are proα2(I) homotrimers (instead of proα1(III) homotrimers which would normally be formed from pro-α chains containing these recognition sequences). Thus according to the invention proα2(I) homotrimers derived from an exogenous source may be formed which do not co-assemble with proα2(I) chains endogenous to the cell in which expression occurs which have "natural" recognition sequences.

In another preferred exogenous manipulation of a gene according to the methods of the invention, the manipulated gene encodes for a molecule comprising at least a first moiety having the activity of a procollagen C-propeptide (i.e. the C-terminal propeptide domain of a pro-α chain) and a second moiety selected from any one of an alien collagen α chain and non-collagen materials, the first moiety being attached to the second moiety. Genes which encode for a second moiety of a non-collagen material (such as those disclosed in PCT/GB96/02122) are examples of pro-α chain derivatives for use according to the invention.

Alternatively the gene or genes may be selected from naturally occurring genes such that the recognition sequence within the C- terminal propeptide domain which is responsible for selective association of pro-α chains such that any pro-α chain expressed from the selected gene will not undesirably co-assemble with pro-α chains endogenously expressed from the host cell into which the gene or genes is or are introduced.

The exogenously selected or modified gene may be incorporated within a suitable vector to form a recombinant vector. The vector may for example be a plasmid, cosmid or phage. Such vectors will frequently include one or more selectable markers to enable selection of cells transfected with the said vector and, preferably, to enable selection of cells harbouring the recombinant vectors that incorporate the exogenously modified gene.

For expression of pro-α chains the vectors should be expression vectors and have regulatory sequences to drive expression of the exogenously modified gene. Vectors not including such regulatory sequences may also be used during the preparation of the exogenously modified gene and arc useful as cloning vectors for the purposes of replicating the exogenously modified gene. When such vectors are used the exogenously modified gene will ultimately be required to be transferred to a suitable expression vector which may be used for production of the pro-α chains.

The system in which the exogenously selected pro-α chain(s) or exogenously manipulated gene or genes of the method of the invention may be expressed and assembled into procollagen may be a cell free *in vitro* system. However it is preferred that the system is a host cell which has been transfected with a DNA molecule according to the second aspect of the invention. Such host cells may be prokaryotic or eukaryotic. Eukaryotic hosts may include yeasts, insect and mammalian cells. Hosts used for expression of the protein encoded by the DNA molecule are ideally stably transformed, although the use of unstably transformed (transient) hosts is not precluded.

Alternatively a host cell system may involve the DNA molecule being incorporated into a transgene construct which is expressed in a transgenic plant or preferably, animal. Transgenic animals which may be suitably formed for expression of such transgene constructs, include birds such as domestic fowl, amphibian species and fish species. Procollagens and / or collagen polymers formed therefrom may be harvested from body fluids or other body products (such as eggs, where appropriate). Preferred transgenic animals are (non-human) mammals, particularly placental mammals. An expression product of the DNA molecule of the invention may be expressed in the mammary gland of such mammals and the expression product may subsequently be recovered from the milk. Ungulates, particularly economically important ungulates such as cattle, sheep, goats, water buffalo, camels and pigs are most suitable placental mammals for use as transgenic animals according to the invention.

Host cells and particularly transgenic plants or animals, may contain other exogenous DNA, the expression of which facilitates the expression, assembly, secretion or other aspects of the biosynthesis of procollagen and even collagen polymers formed therefrom. For example, host cells and transgenic plants or animals may also be manipulated to co-express prolyl 4-hydroxylase, which is a post translation enzyme important in the natural biosynthesis of procollagens, as disclosed in WO-A-9307889.

The methods of the invention enable the expression and assembly of any desired procollagen in a system in which conventionally there would be undesirable co-assembly or hybridisation of pro-α chains. The methods are particularly suitable for allowing the expression of procollagen from a wide variety of cell-lines or transgenic organism without the problems associated with co-assembly with endogenously expressed pro-α chains. A preferred use of the methods of the invention is the production of recombinant procollagens in cell-lines. Examples of cell-lines which may be used are fibroblasts or cell lines derived therefrom. Baby Hamster Kidney cells (BHK cells), Mouse 3T3 cells, Chinese Hamster Ovary cells (CHO cells) and COS cells may be used.

The methods of the invention are particularly useful as an improved means of production of any desired procollagen, particularly for scaled up industrial production by biotechnological means.

The present invention will now be described, by way of example with reference to the accompanying drawings, in which:

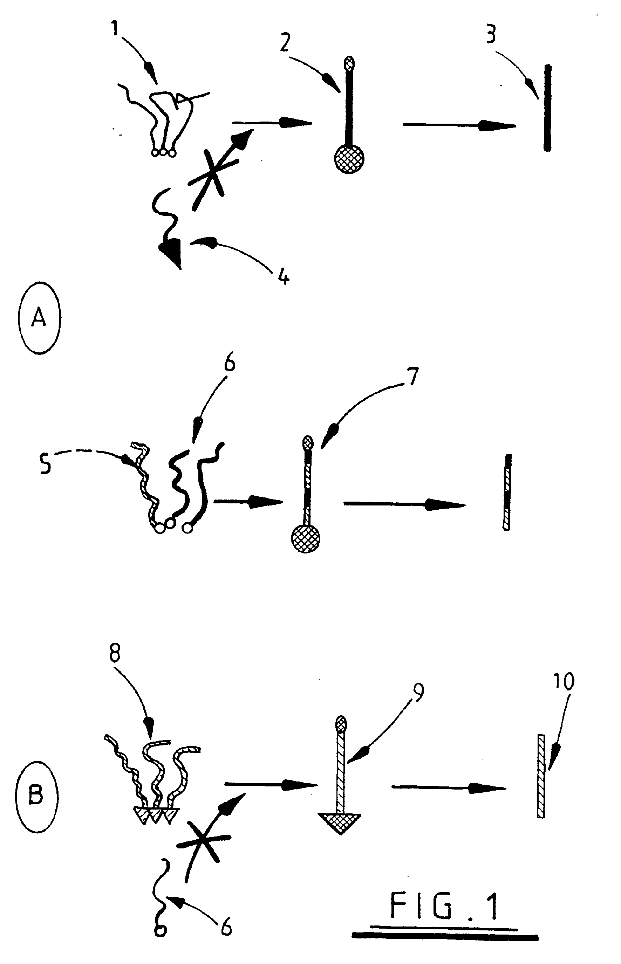

Figure 1 is a schematic representation of the stages in normal procollagen assembly (A) and stages in procollagen assembly according to one embodiment of the invention (B);

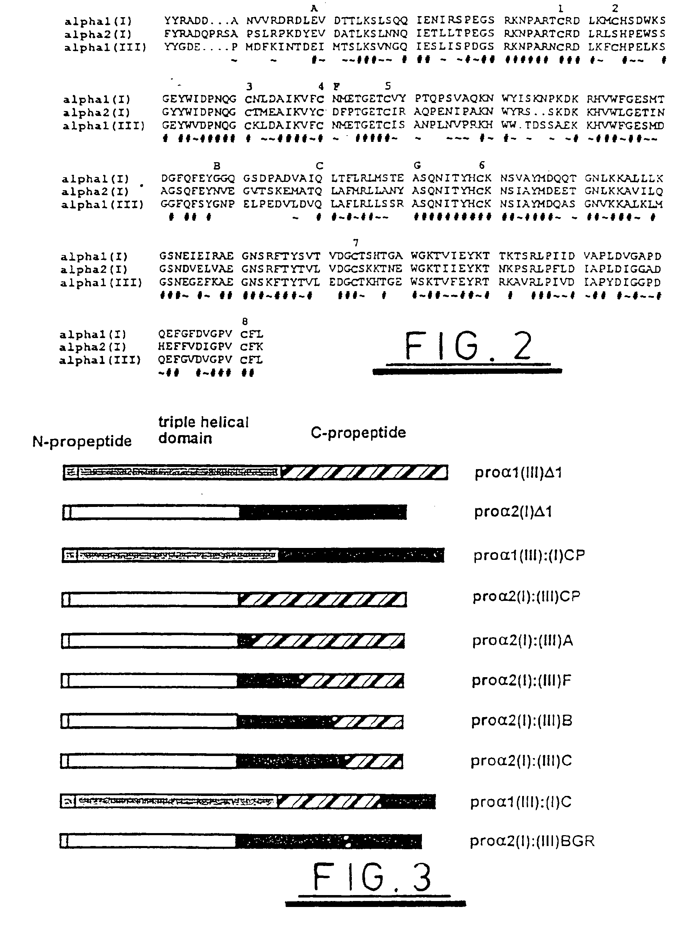

Figure 2 shows an alignment plot of the C- terminal propeptide domains of pro-α chains from type 1 and III collagen. The alignment shows amino acids which are identical (#) or those which are conserved (~). The conserved cysteine residues are numbered 1-8, while letters A, B, C, F, G denote the first amino acid at the junctions between proα1(III) chains and proα2(I) chains of the Example;
Figure 3 is a schematic representation of the chimeric pro-α1 chains described in the Example;

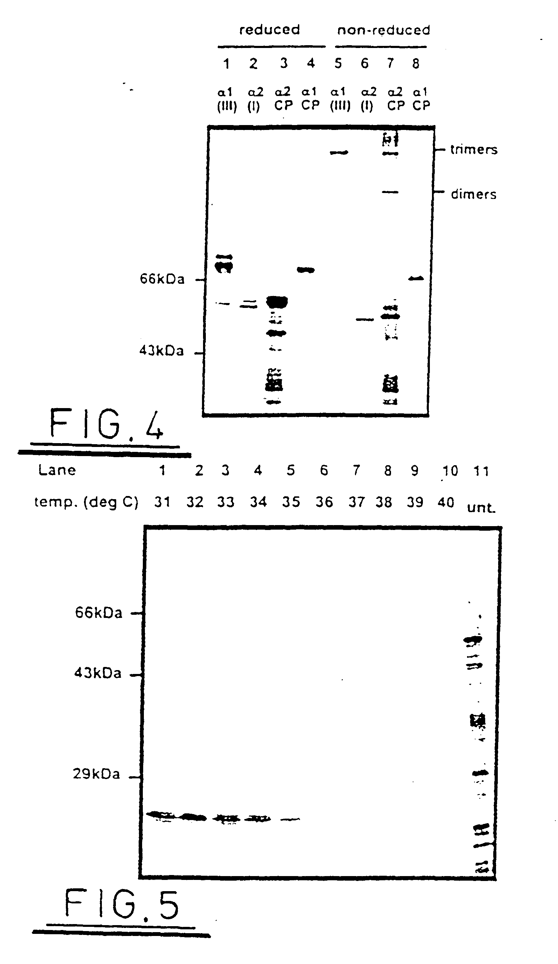

Figure 4 is a photograph of an SDS-PAGE gel, illustrating disulphide bond formation among chimeric gene constructs in which the C-terminal propeptide domain were exchanged, with the following parental and chimeric molecules from the Example run in the indicated lanes of the gel: Proα1 (III)Δ1 [α1(III)], proα2(I)Δ1 [α2(I)] (parental molecule) and proα2(I):(III)CP [α2:CP], proα1(III):(I)CP [α1:CP] (hybrid chains), these molecules were expressed in a rabbit reticulocyte lysate in the presence of semi-permeabilized (SP) HT 1080 cells, after which the SP-cells were isolated by centrifugation, solubilized and the translation products separated by SDS-PAGE through a 7.5% gel under reducing (lanes 1-4) or non-reducing conditions (lanes 5-8);
Figure 5 is a photograph of an SDS-PAGE gel the lanes represent the effect of heat denaturation of proα2(1):(III)CP triple-helix at the specified temperatures, the samples were prepared in the following manner: Proα2(I):(III)CP RNA was translated in the presence of SP-cells, after which the SP-cells were isolated by centrifugation, solubilized and treated with pepsin (100 µg/ml), the reaction mixture was neutralized, diluted in chymotrypsin/trypsin digest buffer and divided into aliquots, each aliquot being heated to a set temperature prior to digestion with a combination of trypsin (100 µg/ml) and chymotrypsin (250 µg/ml), samples were analysed by SDS-PAGE through a 12.5% gel under reducing conditions (lanes 1-10). Lane 11 (unt) contains translation products which have not been treated with proteases;

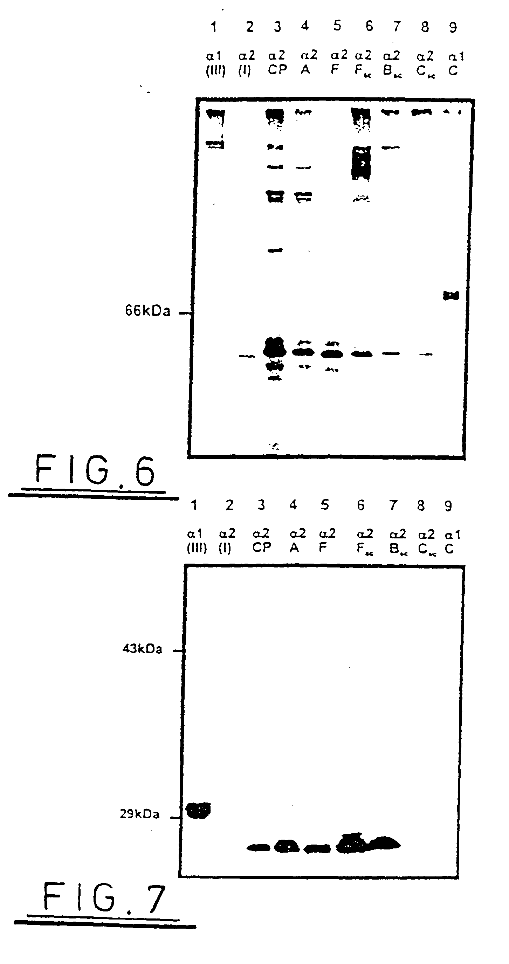

Figure 6 is a photograph of an SDS-PAGE gel illustrating trimerization and triple-helix formation among chimeric procollagen chains, samples were prepared from parental chains proα1(III)Δ1, proα2(I)Δ1 which were made into hybrids proα2(I):(III)CP, A,F,F^{S-C}, Proα1(III):(I)C (α2CP, A.F.F^{S-C},B^{S-C},C^{S-C}, α1C), the hybrids were translated in a rabbit reticulocyte lysate in the presence of SP-cells after which the SP-cells were isolated by centrifugation, solubilized and a portion of the translated material separated by SDS-PAGE under non-reducing conditions through a 7.5% gel (lanes 1-9).
Figure 7 is a photograph of an SDS-PAGE gel illustrating trimerization and triple-helix formation among chimeric procollagen chains, lanes show the remainder of the samples that were loaded on the gel of Fig 6 which were treated with pepsin (100 µg/ml) prior to neutralization and digestion with a combination of trypsin (100 µg/ml) and chymotrypsin (250 µg/ml), the proteolytic digestion products were analysed by SDS-PAGE through a 12.5% gel under reducing conditions (lanes 1-9);

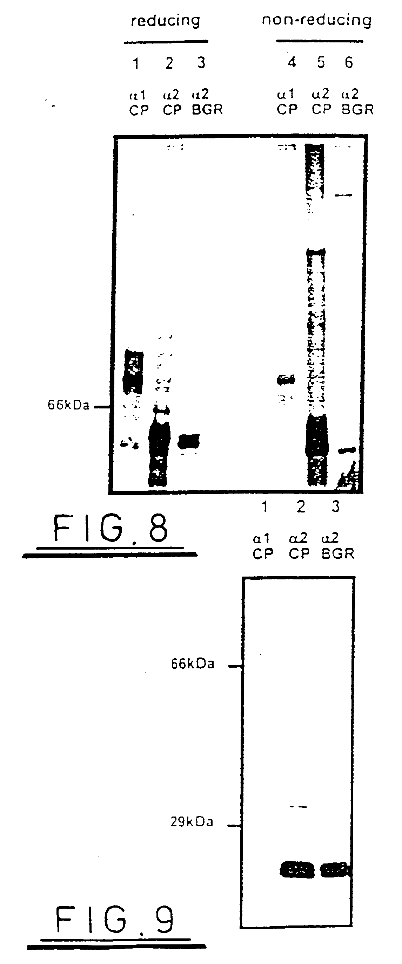

Figure 8 is a photograph of an SDS-PAGE gel, illustrating trimerization and triple-helix formation among chains containing the 23 amino acid B-G motif, the lanes show recombinant procollagen chains proα1(III):(I)CP, proα2(I):(III)CP and proα2(I):(III)BGR^{S-C} which were expressed in a reticulocyte lysate supplemented with SP-cells, after which the SP-cells were isolated by centrifugation, solubilized and a portion of the translated material separated by SDS-PAGE through a 7.5% gel, under reducing (lanes 1-3) of non-reducing conditions (lanes 4-5).
Figure 9 is a photograph of an SDS-PAGE gel, illustrating trimerization and triple-helix formation among chains containing the 23 amino acid B-G motif, the lanes show the remainder of the samples that were loaded on the gel of Figure 9 which were treated with pepsin (100 µg/ml) prior to neutralization and digestion with a combination of trypsin (100 µg/ml) and chymotrypsin (200 µg/ml), the proteolytic digestion products were analysed by SDS-PAGE through a 12.5% gel under reducing conditions (lanes 1-3);

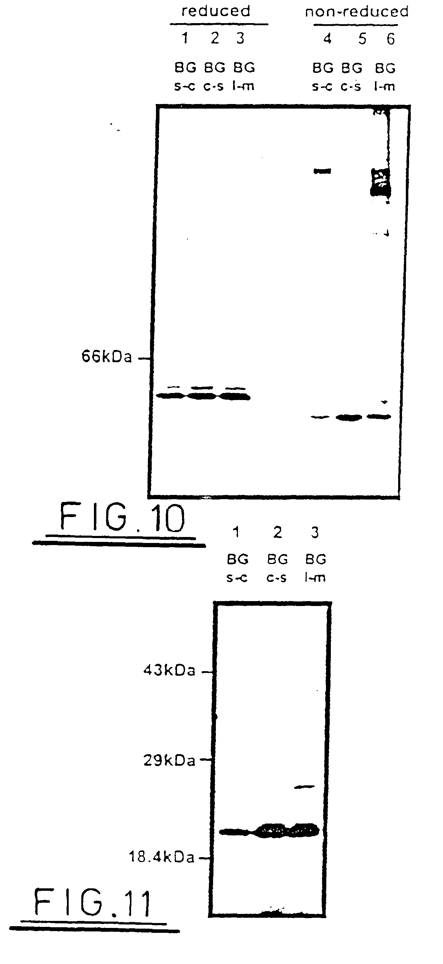

Figure 10 is a photograph of an SDS-PAGE gel, illustrating the effect of Cys-Ser reversion and Leu-Met mutation on the assembly of proα2(I):(III)BGR chains, the lane show recombinant procollagen chains proα2(I):(III)BGR^{S-C} proα2(I):(III)BGR^{C-S}, proα2(I):(III)BGR^{1-m} which were translated in a reticulocyte lysate supplemented with SP-cells after which the cells were isolated by centrifugation, solubilized and a portion of the translated material separated by SDS-PAGE through a 7.5% gel, under reducing (lanes 1-3) or non-reducing conditions (lanes 4-6);
Figure 11 is a photograph of an SDS-PAGE gel, illustrating the effect of Cys-Ser reversion and Leu-Met mutation on the assembly of proα2(I):(III)BGR chains, the lane show the remainder of the samples that were loaded on the gel of Fig 10 which were treated with pepsin (100 µg/ml) prior to neutralization and digestion with a combination of trypsin (100 µg/ml) and a chymotrypsin (250 µg/ml), the proteolytic digestion products were analysed by SDS-PAGE through a 12.5% gel under reducing conditions (lanes 1-3);

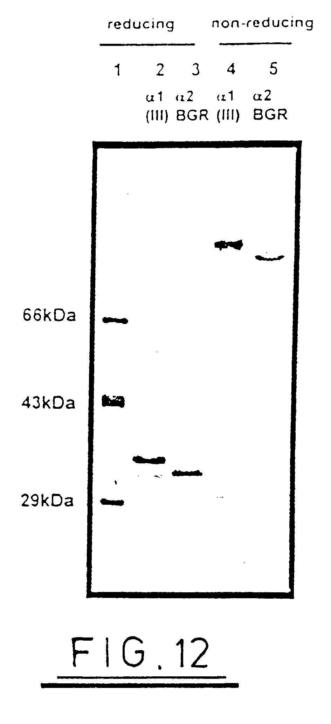

Figure 12 is a photograph of an SDS-PAGE gel, illustrating inter-chain disulfide bonds from between proα2(l):(III)BGR C-terminal propeptide domains, the lanes show recombinant pro-α chains proα1(III)Δ1 and proα2(I):(III)BGR which were translated in a reticulocyte lysate supplemented with SP-cells. The cells were isolated by centrifugation, solubilized and digested with 1.5 units of bacterial collagenase. The products of digestion were analysed by SDS-PAGE through a 10% gel under reducing (lanes 2 and 3) or non-reducing (lanes 4 and 5) conditions; and

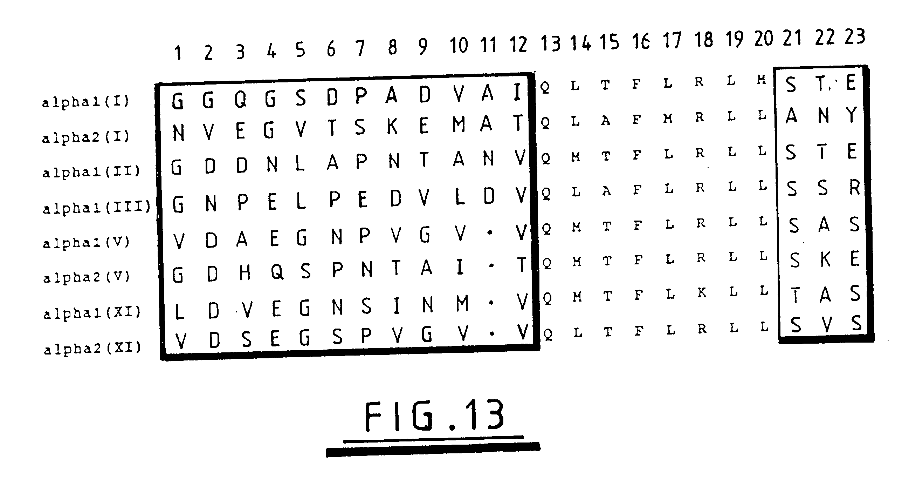

Figure 13 is a schematic representation of sequence alignment of the chain selectivity recognition domains in other fibrillar procollagens, sequence homology within the 23 residue B-G motif is illustrated, the boxed regions indicating the position of the unique 15 residue sub-domain which directs pro-α chain discrimination.

Figure 1 illustrates how procollagen is assembled in the endoplasmic reticulum of a cell. Normally assembly is initiated by type specific association of C-terminal propeptide domains of complimentary pro-α chains (1) to form procollagens (2). Procollagen is secreted from the cell in which it is synthesised and is then acted upon by Procollagen N Proteinases and Procollagen C Proteinases which cleave the N-terminal propeptide and C-terminal propeptide respectively to yield collagen molecules (3). Collagen molecules may then spontaneously aggregate to form collagen fibrils. Pro-α chains with non-complimentary C-terminal propeptide domains (4) do not associate and form procollagens. When exogenous pro-α chains (5) are introduced into a cell they may co-assemble with endogenous pro-α chains (6) which have complimentary C-terminal propeptide domains to form undesirable hybrids (7). According to the methods of the invention exogenously manipulated pro-α chains (8) are generated with C- terminal propeptide domains that are no longer complimentary to the C-terminal propeptide domains of the endogenous pro-α chains (6) such that the exogenously manipulated pro-α chains (8) may form procollagens (9) and subsequently collagen molecules (10) without co-assembly with endogenous pro-α chains (6) occurring.

### EXAMPLE

The inventors generated DNA molecules which may be used according to the methods of the invention. These DNA molecules were used to express pro-α chains with altered selectivity for pro-α chain assembly. Experimental strategy was based on the assumption that transfer of C- terminal propeptide domains (or sequences within the C-propeptide) from the homotrimeric proα1(III) chain to the proα2(I) molecule would be sufficient to direct self-association and assembly into homotrimers of proα2(I). The inventors reconstituted the initial stages in the assembly of procollagen by expressing specific RNAs in a cell-free translation system in the presence of semi-permeabilized cells known to carry out the co- and post-translational modification required to ensure assembly of a correctly aligned triple helix. By analysing the folding and assembly pattern of procollagens formed from a series of chimeric pro-α chains in which specific regions of the C-terminal propeptide domain of proα1 (III) were exchanged with the corresponding region within the proα2(I) chain (and vice versa) the inventors identified a short discontinuous sequence of 15 amino acids within the proα1 (III) C-propeptide which directs procollagen self-association. This sequence is, therefore, responsible for the initial recognition event and is necessary to ensure selective chain association.

### 1. MATERIALS AND METHODS

### 1.1 Construction of recombinant plasmids

pα1(III)Δ1 and pα2(I)Δ1 are recombinant pro-α chains with truncated α chain domains which have been described previously (see Lees and Bulleid (1994) J. Biol. Chem. 269 p24354-243601994). Chimaeric molecules were generated by PCR overlap extension using the principles outlined by Horton (1993) Methods in Molecular Biology Vol 15, Chapter 25, Humana Press Inc., Totowa, NJ. PCRs (100µl) compromised template DNA (500 ng), oligonucleotide primers (100 pmol each) in 10 mM KCI, 20 mM Tris-Hcl pH 8.8, 10mM (NH₄)₂SO₄, 2 mM MgSO₄, 0.1% (v/v) Triton X-100, 300 µM each dNTP. Ten rounds of amplification were performed in the presence of 1 unit Vent DNA polymerase (New England Biolabs, MA). Recombinants pα2(I)Δ1:(III)CP, A, F, S^{s-c}, C^{s-c} were generated using a 5' oligonucleotide primer (5 'AGATGGTCGCACTGGACATC 3') complementary to a sequence 70 bp upstream of an *Sfil* site in pα2(I)Δ1 and a 3' oligonucleotide primer (5' TCGCAGGGATCCGTCGGTCACTTGCACTGGTT 3') complementary to a region 100 bp downstream to the stop codon in pα1(III)Δ1. A *Bam*HI site was introduced into this primer to facilitate subsequent sub-cloning steps. Pairs of internal oligonucleotides, of which one included a 20 nucleotide overlap, were designed to generate molecules with precise junctions as delineated (see Figs 2 and 3) Overlap extension yielded a product of ~990 bp which was purified, digested with *Xho*I-*Bam*HI and ligated into pα2(I)Δ1 from which a 1080 bp *Xho*I*-Bam*HI fragment had been excised. Recombinants pα1(III)Δ1:(I)CP,C were synthesized in a similar manner using a 5' oligonucleotide (5' AATGGAGCTCCTGGACCCATG 3') complementary to a sequence 100bp upstream of an *Xho*I site in a pα(III)Δ1 and a 3' amplification primer (5 'CTGCTAGGTACCAAATGGAAGGATTCAGCTTT 3') which incorporated a *Kpn*I site and was complementary to a region 100bp downstream of the stop codon in pα2(I)Δ1. Overlap extension produced a fragment of 1100 bp which was digested with *Xho*I and *Kpb*I and ligated into pα1(III)Δ from which an 1860 bp fragment had been removed. Recombinant pα2(I):(III)BGR was constructed using the same amplification primer used to synthesize the proα2(I)Δ1:(III) series of chimeras and a 3' oligonucleotide which was identical to that used to generate the proα1(III)Δ1:(I)CP,C constructs except that it contained a *Bam*HI site instead of *Kpn*I (both complementary to pα2(I)Δ1). Primary amplification products were generated from pα2(I)Δ1:(III)B^{s-c} and pα2(I)Δ1 with internal oligonucleotides determining the junction. Overlap extension produced a fragment which was digested with *Sfi*I and *Bam*HI and ligated into pα2(I)Δ1. Site-directed mutagenesis was performed essentially as described by Kunkel *et al*. (Kunkel et al. (1987) Methods in Enzymol. 154 p 367-382), except that extension reactions were performed in the presence of 1 unit T4 DNA polymerase and 1 µg T4 gene 32 protein (Boehringer. Lewes, UK).

### 1.2 Transcription in vitro

Transcription reactions were carried out as described by Gurevich *et al*. (1987) (see Gurevich et al. (1991) Anal. Biochem. 195 p207-213). Recombinant plasmids pα1(II1)Δ1, pα1(III)Δ1:(I)CP,C and pα2(I)Δ1, pα2(I)Δ1:(III)CP, A, F, F^{s-c}, B^{s-c}, C^{s-c} (10µg) were linearized and transcribed using T3 RNA polymerase, or T7 RNA polymerase (Promega, Southampton, UK) respectively. Reactions (100 µl) were incubated at 37°C for 4 h. Following purification over RNeasy columns (Qiagen, Dorking, UK), RNA was resuspended in 100 µl RNasefree water containing 1 mM DTT and 40 units RNasin (Promega, Southampton, UK).

### 1.3 Translation in vitro

RNA was translated using a rabbit reticulocyte lysate (FlexiLysate, Promega, Southampton) for 2 hours at 30°C in the absence of exogenous DTT. The translation reaction (25µl) contained 17µl reticulocyte lysate, 1 µl 1 mM amino acids (minus methionine), 0.45 µl 100mM KCI, 0.25 µl ascorbic acid (5 mg/ml), 15 µCi [B-³⁵S]methionine (Amersham International, Bucks, UK), 1 µl transcribed RNA and 1 µl (~2 x 10⁵) semi-permeabilized cells (SP-cells) prepared as described by Wilson et al. (1995) Biochem. J. 307 p679-687. After translation, *N*-ethylmaleimide was added to a final concentration of 20 mM. SP-cells were isolated by centrifugation in a microfuge at 10000 g for 5min and the pellet resuspended in an appropriate buffer for subsequent enzymic digestion or gel electrophoresis.

### 1.4 Bacterial collagen ase digestion

SP-cells were resuspended in 50 mM Tris-HCl pH 7.4 containing 5 mM CaCl₂, 1 mM phenylmethanesulfonyl fluoride (PMSF), 5mM *N*-ethylmaleimide and 1% (v/v) Triton X-100 and incubated with 3 units collagenase form III (Advance Biofacture, Lynbrook, NJ) and incubated at 37°C for 1h. The reaction was terminated by the addition of SDS-PAGE sample buffer.

### 1.5 Proteolytic digestion

Isolated SP-cells were resuspended in 0.5% (v/v) acetic acid, 1% (v/v) Triton X-100 and incubated with pepsin (100 µg/ml) for 2 h at 20°C or 16 h at 4°C. The reactions were stopped by neutralization with Tris-base (100 mM). Samples were then digested with a combination of chymotrypsin (250 µg/ml) and trypsin (100 µg/ml) (Sigma, Poole, Dorset, UK) for 2 min at room temperature in the presence of 50 mM Tris-HCI pH 7.4 containing 0.15 M NaCl, 10 mM EDTA. The reactions were stopped by the addition of soy bean trypsin inhibitor (Sigma, Poole, Dorset, UK) to a final concentration of 500 µg/ml and boiling SDS-PAGE loading buffer. Samples were then boiled for 5 min.

### 1.6 Thermal denaturation

Pepsin-treated samples were resuspended in 50 mM Tris-HCI pH 7.4 containing 0.15 M NaCl, 10mM EDTA. and aliquots placed in a thermal cycler. A stepwise temperature gradient was set up from 31°C to 40°C with the temperature being held for 2 min at 1°C intervals. At the end of each time period the sample was treated with a combination of chymotrypsin, as described above.

### 1. 7 SDS-PA GE

Samples resuspended in SDS-PAGE loading buffer (0.0625 M Tris-HCI pH 6.8, SDS (2% w/v), glycerol (10% v/v) and Bromophenol Blue) in the presence or absence of 50 mM DTT and boiled for 5 min. SDS-PAGE was performed using the method of Laemmli (1970) Nature 227 p680-685. After electrophoresis, gels were processed for autoradiography and exposed to Kodak X-Omat AR film, or images quantified by phosphoimage analysis.

### 2. RESULTS

### 2.1 Transfer of the proα1(III) C-propeptide to the proα(I)2 chain is sufficient to direct self-assembly.

Experimental strategy was based on the assumption that transfer of the C-terminal propeptide domain from the proα1(III) chain to the proα2(I) chain should be sufficient to direct self-recognition and assembly into homotrimers. Hence, by exchanging different regions within the proα1(III) C- terminal propeptide domain with the corresponding sequence from the proα2(I) chain the intention was to distinguish between sequences that direct the folding of tertiary structure and those involved in the selection (i.e. recognition of pro-α chains) process. To simplify analysis of the translation products chimeric procollagen molecules were constructed from two parental procollagen 'mini-chains', proα1(III)Δ1 and proα(I)Δ1. These molecules, which have been described previously (Lees and Bulleid, 1994), comprise both the N- and C- terminal propeptides domains together with truncated triple-helical domains. The initial assumption was tested by analysing the folding and assembly of chimeric procollagen chains in which the C-terminal propeptide domain of the proα2(I) chain was substituted with the equivalent domain from the proα1(III)Δ1 chain (proα2-(I):(III)CP) and, conversely, where the C-propeptide of proα1(III) chain was replaced with that from proα2(I)Δ1 chain (proα1(III):(I)CP) (see Figs 2 and 3). The C-propeptide (CP) junction points were determined by the sites of cleavage by the procollagen C-proteinase (PCP) which is known to occur between Ala and Asp (residues 1119-1120) in the proα2(I) chain (Kessler (1996) Science 271 p360-362). In the absence of data regarding the precise location of cleavage within the proα(III) chain, the inventors chose to position the junction between Ala and Pro (residues 1217-1218). However, Kessler and co-workers (1996) have subsequently shown that cleavage by PCP occurs between Gly and Asp (residues 1222-1223), with the consequence that recombinant proα2(I):(III)CP includes an additional four residues derived from the proα(III) C-telopeptide, whilst the C-telopeptide in construct proα1(III):(I)CP is missing those same four amino acids. RNA transcripts were transcribed *in vitro* and expressed in a cell-free system comprising a rabbit reticulocyte lysate optimized for the formation of disulfide bonds supplemented with semi-permeabilized HT 1080 cells (SP-cells), which has been shown previously to carry out the initial stages in the folding, post-translational modification and assembly of procollagen (Bulleid et al., (1996) Biochem. J. 317 p195-202). The C-terminal propeptide domains of both proα1(III) and proα2(I) chains contain cysteine residues which participate in the formation of interchain disulfide bonds. Translation products were, therefore, separated by SDS-PAGE under reduced and non-reduced conditions in order to detect disulfide-bonded trimers. Translation of the parental molecules proα1(III)Δ1 and proα2(I)Δ1 yielded major products of ~ 77 kDa and 61 kDa respectively (Figure 4, lanes 1 and 2), the size differential being accounted for by the relative molecular weights of the N-propeptides and truncated triple-helical domains in each molecule (Lees and Bulleid, 1994). The heterogeneity of the translation products is due to hydroxylation of proline residues in the triple-helical domain that leads to an alteration in electrophoretic mobility (Cheah et al., (1979) Biochem. Biophys. Res. Comm. 91 p1025-1031). The additional lower molecular weight proteins present in lanes 3 and 7 probably represent translation products obtained after initiation of translation at internal start codons. We have previously shown that these minor translation products are not translocated into the endoplasmic reticulum (Lees and Bulleid, 1994). The presence of high molecular weight species under non-reducing conditions but not reducing conditions is indicative of interchain disulfide bond formation. Separation under non-reduced conditions revealed that proα1(III)Δ1, but not proα(I)Δ1, chains were able to self-associate to form disulfide-bonded trimers (Figure 4, lanes 5 and 6). A similar examination of chimeric chains proα2(I):(III)CP and proα1(III):(I)CP revealed that only proα2(I):(III)CP chains were able to form disulfide-bonded homotrimers (Figure 4, lanes 3, 4, 7 and 8) demonstrating that the C-propeptide from type III procollagen is both necessary and sufficient to drive the initial association between procollagen chains.

It has been shown previously that proα1(III)Δ1 chains synthesised in the presence of SP-cells were resistant to a combination of pepsin, chymotrypsin and trypsin in a standard assay used specifically to detect triple-helical procollagen (Bulleid *et al.,* 1996). The inventors confirmed that proα2(I):(III)CP chains had the ability to form a correctly aligned triple-helix by performing a thermal denaturation experiment in which translated material was heated to various temperatures prior to protease treatment (Figure 5). The results indicate that at temperatures below 35°C a protease-resistant triple-helical fragment is present, but at temperatures above 35°C the triple-helix melts and becomes protease sensitive (Figure 5, lanes 1-10). The melting temperature (*T*ₘ) was calculated to be ~35.5°C after quantification by phophorimage analysis. The *T*ₘ value obtained for proα2-(I):(III)CP is significantly lower than the figure of 39.5°C obtained for proα1(III)Δ1 (Bulleid *et al.,* 1996) and probably reflects the percentage of hydroxyproline residues relative to the total number of amino acids in the triple-helical domain (11% and 15% respectively). These results indicate that transfer of the proα(III) C-propeptide enables the inventors to generate an entirely novel procollagen species comprising three proα2(I) chains that fold into a correctly aligned triple-helix.

### 2.2 Assembly of recombinant procollagen chains with chimeric C-propeptides.

Given that the proα2(I):(III)CP hybrid pro-α chain includes all of the information required for self-association we reasoned that progressive removal of the proα1(III) C-propeptide sequence and replacement with the corresponding proα2(I) sequence would eventually disrupt the chain selection mechanism. Conversely, it is anticipated that transfer or progressively more proα1(III) C-terminal propeptide domain sequence to the proα1(III):(I)CP chimeric chain would yield a molecule which was capable of self-assembly. A series of procollagen chains with chimeric C-terminal propeptide domains was constructed and the ability of individual chains to form homotrimers with stable triple-helical domains was assessed. A schematic representation of these recombinants is presented in Figure 2, with the letters A, B, C, F and G denoting the position of each junction. It should be noted that the proα 1(III) and proα2(I) C-propeptides differ in their complement of cysteine residues, with proα2(I) lacking the Cys2 residue. Our previous data suggest that interchain disulfide bond within the C-propeptide of type III procollagen form exclusively between Cys2 and 3 (Lees and Bulleid, 1994). However, interchain disulfide bonding, between either the C-terminal propeptide domains to C-telopeptides is not required for chain association and triple-helix formation (Bulleid *et al.,* 1996), therefore, it is possible that homotrimers may form between chimeric pro-α chains which lack either the C-terminal propeptide domain Cys2 residue or the C-telopeptide cysteine [only found in the triple-helical domain of proα1(III)]. These molecules will not, however, contain interchain disulfide bonds and, as a consequence will not appear as oligomers after analysis under non-reducing conditions. To circumvent this problem, where appropriate, the inventors generated their hybrid chains from a recombinant proα2(I)Δ1^{s-c} (Lees and Bulleid, 1994) in which the existing serine residue was substituted for cysteine, thus restoring the potential to form trimers stabilized by interchain disulfide bonds. It should also be noted that whilst proα1(III):(I)CP lacks Cys2, it does still retain the potential to form disulfide-bonded trimers by virtue of the two cysteine residues located at the junction of the triple-helical domain and the C-telopeptide, Parental chains proα2(I)Δ1 and hybrids proα2(I):(III)CP, A, F, F^{s-c}, B^{s-c}, C^{s-c}, proα1(III):(I)C were translated in the presence of SP-cells and the products separated by SDS-PAGE under non-reducing conditions (Figure 6). The results demonstrate that recombinants proα1(III)Δ1, proα2(I):(III)CP, A, F^{s-c}, B^{s-c} (Figure 6. lanes 1, 3, 4, 6 and 7) are able to form interchain disulfide-bonded trimers and dimers while proα1(III)Δ1, proα2(I):(III)F, C^{s-c} and proα1(III):(I)C (Figure 6, lanes 2, 5, 8 and 9) remain monomeric. We have already demonstrated that interchain disulfide bonding is not a prerequisite for triple-helix formation (Bulleid *et al.,* 1996), therefore, the inability to form disulfide-bonded trimers does not preclude the possibility that the molecules assemble to form a triple-helix. To ascertain whether the chimeric chains had the ability to fold into a correctly aligned triple-helix, we treated translation products with a combination of pepsin, chymotrypsin and trypsin and analysed the digested material under reducing conditions by SDS -PAGE. As shown in Figure 7, recombinants proα1(III)Δ1, proα2(I):(III)CP, A, F^{s-c}, F, B^{s-c} (Figure 7, lanes 1, 3, 4, 5, 6 and 7) all yielded protease-resistant fragments. The size differential reflects the relative lengths of the triple-helical domains in each of the parental molecules [proα2(I)Δ1-185 residues and proα1(III)Δ1-192 residues]. The ability of proα2(I):(III)F to form a stable triple-helix confirms that interchain disulfide bonding is not necessary for triple-helix folding. Thus, hybrid molecules containing sequences from the proα2 C-terminal propeptide domains between the propeptide cleavage site and the B-junction are able to form homotrimers with stable triple-helical domains and, therefore, contain all of the information necessary to direct chain self-assembly. These results indicate that the signal(s) which controls chain selectivity must be located between the B-junction and the C-terminus of the C-propeptide. Neither proα2(I):(III)C^{s-c} nor proα1(III):(I)C chains are able to fold into a triple helix. The inability of these reciprocal constructs to self-associate suggests that chain selectivity is mediated. either by a co-linear sequence that spans the C-junction or by discontinuous sequence domains located on either side of the C-junction.

### 2.3 Identification of a sequence motif from the proα1(III) C-propeptide which directs chain self-assembly

Procollagen chain selectivity is probably mediated through one or more of the variable domains located within the C-terminal propeptide domain. The sequence between the B- and C-junctions is one of the least conserved among the procollagen C-propeptides (Figure 2), yet to inventors have demonstrated that inclusion of this domain, in the absence of proα1(III) sequence distal to the C-junction, is not sufficient to direct chain assembly. To ascertain whether the recognition sequence for chain recognition had indeed been interrupted a further recombinant, proα2(I):(III)BGR^{s-c} (B-G replacement) was generated, which contained all of the proα(I)Δ1 sequence apart from the Ser→Cys mutation at Cys2 and a stretch of 23 amino acids derived from the type III C-propeptide which spans the C-junction from points B to G, the B-G motif: ^{b}GNPELPEDVLDV^{c}QLAFLRLLSSR^{g} (underscoring indicates the most divergent residues, see Figure 2). The location of the G-boundary in the replacement motif allowed for the inclusion of the first non-conserved residues after the C-junction (SR). When expressed in the presence of SP-cells the chimeric proα2(I):(III)BGR^{s-c} chains were able to form inter-chain disulfide-bonded molecules (Figure 8, lane 6) demonstrating that the C-terminal propeptide domains were capable of self-association. Furthermore, this hybrid was able to fold and form a stable triple-helix as judged by the formation of a protease-resistant fragment (Figure 9, lane 3). Proα2(I):I):(III)BGR^{s-c} contains a Ser→Cys substitution which enabled the inventors to assay for the formation of disulfide-bonded trimers. Previous data demonstrated that this substitution alone does not enable wild-type proα2(I)Δ1 claims to form homotrimers (Lees and Bulleid, 1994). Nevertheless, to eliminate the possibility that this mutation influences the assembly pattern a revertant proα(I):(III)BGR^{c-s} which contains the wild-type complement of Cys residues was created. As expected proα2(I):(III)BGR^{c-s} was unable to form disulfide-bonded trimers (Figure 10, lane 5) but did assemble correctly into a protease-resistant triple helix (Figure 11, lane 3). Thus, the 23-residue B-G motif contains all of the information required to direct procollagen self-assembly.

The ability of the proα2(I):(III)BGR^{s-c} chains to form interchain disulfide bonds suggests that this molecules is able to associate via its C-propeptide. However, to confirm that this is indeed the case the inventors carried out a collagenase digestion of the products of the translation (Figure 12). Bacterial collagenase specifically digests the triple-helical domain, leaving both the N- and C- propeptides intact. The N-propeptides of both chains do not contain any methionine residues and as a consequence, the only radio labelled product remaining after digestion is the C-propeptide. Comparison of the samples separated under reducing and non-reducing conditions demonstrated that inter-chain disulfide-bonded trimers were formed within the C-terminal propeptide domains of proα1(III)Δ1 and proα2(I):(III)BGR^{s-c} chains (Figure 12, lanes 2 and 4, and 3 and 5). This demonstrates that these chains do indeed associate via their C-terminal propeptide domains.

### 2.4 The effect of Leu→Met substitution on proα2(I):BGR assembly

Analysis of the 23 amino acid B-G motif from the proα1(III) and proα2(I) chains (Figure 13) indicates that residues 13-20 (QLAFLRLL) are identical with the exception of position 17, Leu (L) in proα1(III) and Met (M) in proα2(I). Using site-directed mutagenesis the inventors substituted the existing Leu residue with Met to create proα2(I):(III)BGR^{1-m} and monitored the effect of this mutation on chain assembly. The Leu→Met mutagenesis was performed using recombinant proα(I):(III)BGR^{s-c} and proα2(I):(III)BGR^{1-m} and were able to form interchain disulfide-bonded molecules when analysed under non-reducing conditions (Figure 10, lanes 4 and 6). Both constructs formed protease-resistant triple-helical domains (Figure 11, lanes 1 and 3). The Leu→Met substitution did not, therefore, disrupt the process of chain selection nor did it prevent the formation of a correctly aligned triple-helix. These observations lead to the conclusion that a discontinuous sequence of 15 amino acids: (GNPELPEDVLDV......SSR) contains all of the information necessary to allow procollagen chains to discriminate between each other and assemble in a type-specific manner.

### 3. DISCUSSION

The molecular mechanism which enables closely related procollagen chains to discriminate between each other is a central feature of the assembly pathway. The initial interaction between the C-terminal propeptide domains both ensures that the constituent chains are correctly aligned prior to nucleation of the triple-helix and propagation in a C- to N- direction, and that component chains associate in a collagen type-specific manner. As a consequence, recognition signals which determine chain selectivity are assumed to reside within the primary sequence of this domain, presumably within a region(s) of genetic diversity. By generating chimeric procollagen molecules from parental 'mini-chains' proα1(III)Δ1 and proα2(I)Δ1 the inventors have demonstrated that transfer of the proα1(III) C-terminal propeptide domain to the naturally hetrotrimeric proα2(I) molecule was sufficient to direct formation of homotrimers. Furthermore, analysis of a series of molecules in which specific sequences were interchanged from proα1(III) and proα2(I) C-terminal propeptide domains allowed the inventors to identify a discontinuous sequence of 15 amino acids (GNPELPEDVLDV.......SSR) within the proα1(III) C-propeptide, which, if transferred to the corresponding region within the proα1(III) recognition motif to the proα2(I) chain did not appear to have an adverse effect on chain alignment, allowing the triple-helical domains to fold into a protease-resistant confirmation. This sequence motif is, therefore, both necessary and sufficient to ensure that procollagen chains discriminate between each other and assemble in a type-specific manner.

In order to establish a structure-function relationship for the chain recognition domain, the inventors examined the hydropathy profile and secondary structure potential of the 23-residue B-G sequence : GNPELPEDVLDVQLAFLRLLSSR. The data indicate that the 15-residue chain recognition motif: GNPELPEDVLDV....SSR is markedly hydrophilic, in contrast to the hydrophobic properties of the conserved region: QLAFLRLLL. These features are entirely consistent with a potential role for this motif in mediating the initial association between the component procollagen monomers. An examination of the 15-residue recognition motif from other fibrillar procollagens predicts that they are all relatively hyrophilic and probably assume a similar structural conformation, regardless of the degree of diversity in the primary sequence (Figure 13). It is, presumably, the nature of the amino acids changes which provides the distinguishing topographical features necessary to ensure differential chain association. An examination of the B-G sequence alignment (Figure 13) indicates that residues 1, 2, 12 and 21 are more tightly conserved that amino acids 3-11, 22 and 23, suggesting that the latter may form a core recognition sequence that is of critical importance in the selection process. We do not know whether the other four residues participate directly in chain discrimination but this can be tested experimentally by site-directed mutagenesis.

The inventors have identified the functional domain which determines chain selectivity and show that trimerization is initiated via an interaction(s) between these identified recognition sequences. It is unclear, however, whether the interactions which determine chain composition are the same as those which allow productive association and stabilization of the trimer. The nature of potential stabilizing interactions is uncertain, but recent data (Bulleid *et al.,* 1996) indicate that, for type III procollagen at least, the formation of interchain disulfide bonds does not play a direct role in procollagen assembly. It has also been postulated that a cluster of four aromatic residues, which are conserved in the fibrillar collagens, collagens X, VIII and collagen like complement factor C1q, may be of strategic importance in trimerization.

The C-telopeptides were originally proposed to have a role in both procollagen assembly and in chain discrimination, the latter by virtue of the level of sequence diversity between various procollagen chains. However, the inventors have recently demonstrated (Bulleid *et al.,* 1996) that the C-telopeptides of type III collagen do not interact prior to nucleation of the triple-helix, ruling out a role for this peptide sequence in the initial association of the C-propeptides. Data obtained from the assembly of hybrid chains indicates that the ability to discriminate between chains does not segregate with the species of C-telopeptide, lending support to this assertion.

Using this approach the inventors have been able to synthesize an entirely novel procollagen species compromising three proα2(I)Δ1 chains [proα2(I)Δ1]₃. Throughout this study procollagen 'mini-chains' with truncated triple-helical domains were used; however, the inventors have also demonstrated that full-length proα2(I) chains containing the 15-residue proα1(III) recognition sequence also self-associate into a triple-helical conformation (data not shown). Thus, the ability to introduce the chain recognition sequence into different pro-α chains provides the means to design novel collagen molecules with defined chain compositions. This, in turn, introduces the possibility of producing collagen matrices with defined biological properties, such as enhanced or differential cell-binding or adhesion properties. Furthermore, the identification of a short peptide sequence which directs the initial association between procollagen chains may provide a target for therapeutic intervention allowing for the modulation or inhibition of collagen deposition.

The chimeric constructs described above may be used in the method of the present invention to allow the expression of exogenous procollagens in any cell-line without the problems associated with co-assembly with endogenously expressed procollagen. The uses of the methods of the invention are to express procollagen in cells grown in culture. This will be of particular relevance for the production of recombinant procollagen in cell-lines such as fibroblasts which normally efficiently synthesis fibrillar collagens and in the treatment of collagen diseases by gene therapy.

## Claims

1. A method of producing a first procollagen comprising expressing, in a system which co-expresses and assembles at least one second procollagen, exogenously selected or exogenously manipulated genes which express pro-α chains thereof with domains which have the activity of C-terminal propeptide domains for assembly into the first procollagen but which will not co-assemble with the C- terminal propeptide of the pro-α chains thereof that assemble to form the said at least one second procollagen, said genes including one which has been exogenously manipulated so as to express a pro-α chain which comprises
(i) a first moiety having the activity of a C-terminal propetide domain and incorporating a recognition sequence of a first type pro-α chain conferring a selectivity on the assembly of the pro-α chain into a procollagen; and
(ii) a second moiety containing a triple helix forming domain from a pro-α chain different from said first type,
said first moiety being attached to said second moiety so that said recognition sequence permits co-assembly of said pro-α chain for assembly into said first procollagen with other pro-α chains having said activity and a triple helix forming domain, whereby said first procollagen is produced, said method being other than a method of therapeutic treatment.

2. The method according to claim 1, wherein the recognition sequence is the amino acid sequence GGQGSDPADV AlQLTFLRLM STE.

3. The method according to claim 1, wherein the recognition sequence is the amino acid sequence NVEGVTSKEM ATQLAFMRLL ANY.

4. The method according to claim 1, wherein the recognition sequence is the amino acid sequence GDDNLAPNTA NVQMTFLRLL STE.

5. The method according to claim 1, wherein the recognition sequence is the amino acid sequence GNPELPEDVL DVQLAFLRLL SSR.

6. The method according to claim 1, wherein the recognition sequence is the amino acid sequence VDAEGNPVGV VQMTFLRLL SAS.

7. The method according to claim 1, wherein the recognition sequence is the amino acid sequence GDHQSPNTAI TQMTFLRLL SKE.

8. The method according to claim 1, wherein the recognition sequence is the amino acid sequence LDVEGNSINM VQMTFLKLL TAS.

9. The method according to claim 1, wherein the recognition sequence is the amino acid sequence VDSEGSPVGV VQLTFLRLL SVS.

10. The method according to any one of claims I to 9, wherein the exogenously manipulated gene encodes for pxo-α chains formed from combinations of fragments of pro-α1 (I), pro-α2 (I), pro-α1 (II), pro-α1 (III), pro-α1 (V), pro-α2 (V), pro-α1 (XI) or pro-α2 (XI) pro-α chains.

11. The method according to claim 10, wherein the exogenously manipulated gene encodes a modified proα2(I) chain in which the recognition sequence of the proα2(I) chain has been substituted by the recognition sequence of a proα1(III) chain.

12. The method according to any preceding claim, wherein the gene is incorporated within a vector.

13. The method according to claim 12, wherein the vector is a plasmid, cosmid or phage.

14. The method according to any preceding claim, wherein the system is a host cell transfected with the gene.

15. The method according to claim 14, wherein the host cell is eukaryotic.

16. The method according to claim 15, wherein, the host cell is a yeast, insect or mammalian cell.

17. The method according to claim 16, wherein the host cell is a mammalian cell and selected from fibroblasts or cell lines derived therefrom, Baby Hamster Kidney cells, Mouse 3T3 cells, Chinese Hamster Ovary cells or COS cells.

18. The method according to any one of claims 1 - 14, wherein the system is a transgenic plant or non-human animal.

19. The method according to claim 18, wherein the system is a transgenic animal and is a non-human placental mammal.

20. The method according to claim 19, wherein the placental mammal is any one of cattle, sheep, goats, water buffalo, camels or pigs.

## Patentansprüche

1. Verfahren zur Herstellung eines ersten Procollagens, wobei das Verfahren aufweist: Expression in einem System, das mindestens ein zweites Procollagen koexprimiert und assembliert, von exogen selektierten oder exogen manipulierten Genen, die Pro-α-Ketten mit Domänen exprimieren, welche die Aktivität von C-terminalen Propeptid-Domänen für die Assemblierung in das erste Procollagen aufweisen, aber nicht mit dem C-terminalen Propeptid der Pro-α-Ketten koassemblieren, die zur Bildung des mindestens einen zweiten Procollagens assemblieren, wobei zu den Genen ein Gen gehört, das exogen manipuliert worden ist, um eine Pro-α-Kette zu exprimieren, die aufweist:
(i) eine erste Komponente, welche die Aktivität einer C-terminalen Propeptid-Domäne aufweist und Erkennungssequenz einer Pro-α-Kette von einem ersten Typ enthält, die der Assemblierung der Pro-α-Kette in ein Procollagen Selektivität verleiht; und
(ii) eine zweite Komponente, die eine Tripelhelix-bildende Domäne aus einer anderen Pro-α-Kette als dem ersten Typ enthält,
wobei die erste Komponente so an die zweite Komponente angelagert ist, dass die Erkennungssequenz eine Koassemblierung der Pro-α-Kette für die Assemblierung in das erste Procollagen mit anderen Pro-α-Ketten zulässt, welche die Aktivität und eine Tripelhelix-bildende Domäne aufweisen, wodurch das erste Procollagen erzeugt wird, wobei das Verfahren keine therapeutsiche Behandlungsmethode ist.

2. Verfahren nach Anspruch 1, wobei die Erkennungssequenz die Aminosäuresequenz GGQGSDPADV AIQLTFLRLM STE ist.

3. Verfahren nach Anspruch 1, wobei die Erkennungssequenz die Aminosäuresequenz NVEGVTSKEM ATQLAFMRLL ANY ist.

4. Verfahren nach Anspruch 1, wobei die Erkennungssequenz die Aminosäuresequenz GDDNLAPNTA NVQMTFLRLL STE ist.

5. Verfahren nach Anspruch 1, wobei die Erkennungssequenz die Aminosäuresequenz GNPELPEDVL DVQLAFLRLL SSR ist.

6. Verfahren nach Anspruch 1, wobei die Erkennungssequenz die Aminosäuresequenz VDAEGNPVGV VQMTFLRLL SAS ist.

7. Verfahren nach Anspruch 1, wobei die Erkennungssequenz die Aminosäuresequenz GDHQSPNTAI TQMTFLRLL SKE ist.

8. Verfahren nach Anspruch 1, wobei die Erkennungssequenz die Aminosäuresequenz LDVEGNSINM VQMTFLKLL TAS ist.

9. Verfahren nach Anspruch 1, wobei die Erkennungssequenz die Aminosäuresequenz VDSEGSPVGV VQLTFLRLL SVS ist.

10. Verfahren nach einem der Ansprüche 1 bis 9, wobei das exogen manipulierte Gen für Pro-α-Ketten codiert, die aus Fragmentkombinationen von Pro-α1(I)-, Pro-α2(I)-, Pro-α1(II)-, Pro-α1(III)-, Pro-α1(V)-, Pro-α2(V)-, Pro-α1(XI)- oder Pro-α2(XI)-Ketten gebildet werden.

11. Verfahren nach Anspruch 10, wobei das exogen manipulierte Gen für eine modifizierte Pro-α2(I)-Kette codiert, in der die Erkennungssequenz der Pro-α2(I)-Kette durch die Erkennungssequenz einer Pro-α1(III)-Kette substituiert worden ist.

12. Verfahren nach einem der vorstehenden Ansprüche, wobei das Gen in einen Vektor eingebaut ist,

13. Verfahren nach Anspruch 12, wobei der Vektor ein Plasmid, Cosmid oder Phage ist.

14. Verfahren nach einem der vorstehenden Ansprüche, wobei das System eine mit dem Gen transfizierte Wirtszelle ist.

15. Verfahren nach Anspruch 14, wobei die Wirtszelle eukaryotisch ist.

16. Verfahren nach Anspruch 15, wobei die Wirtszelle eine Hefe-, Insekten- oder Säugetierzelle ist.

17. Verfahren nach Anspruch 16, wobei die Wirtszelle eine Säugetierzelle und unter Fibroblasten oder daraus abgeleiteten Zalllinien, Baby-Hamsternierenzellen, Maus-3T3-Zellen, Ovarialzellen des chinesischen Hamsters oder COS-Zellen ausgewählt ist.

18. Verfahren nach einem der Ansprüche 1-14, wobei das System eine transgene Pflanze oder ein transgenes nichtmenschliches Tier ist.

19. Verfahren nach Anspruch 18, wobei das System ein transgenes Tier und ein nichtmenschliches plazentales Säugetier ist.

20. Verfahren nach Anspruch 19, wobei das plazentale Säugetier zu Rindern, Schafen, Ziegen, Wasserbüffeln, Kamelen oder Schweinen gehört.

## Revendications

1. Procédé de production d'un premier procollagène, comprenant exprimer, dans un système qui co-exprime et assemble au moins un deuxième procollagène, des gènes sélectionnés d'une manière exogène ou manipulés d'une manière exogène, qui expriment des chaînes pro-α de celui-ci avec des domaines qui ont l'activité des domaines de propeptide C-terminal pour l'assemblage dans le premier procollagène, mais qui ne se co-assembleront pas avec le propeptide C-terminal des chaînes pro-α de celui-ci qui s'assemblent pour former ledit au moins un deuxième procollagène, lesdits gènes en incluant un qui a été manipulé d'une manière exogène afin d'exprimer une chaîne pro-α qui comprend:
(i) une première fraction ayant l'activité d'un domaine de propeptide C-terminal et incorporant une séquence de reconnaissance d'une chaîne pro-α d'un premier type conférant une sélectivité à l'assemblage de la chaîne pro-α dans un procollagène; et
(ii) une deuxième fraction contenant un domaine formant une triple hélice à partir d'une chaîne pro-α différente dudit premier type,
ladite première fraction étant attachée à ladite deuxième fraction de sorte que ladite séquence de reconnaissance permet le co-assemblage de ladite chaîne pro-α pour l'assemblage dans ledit premier procollagène avec d'autres chaînes pro-α ayant ladite activité et un domaine formant une triple hélice, en vertu de quoi ledit premier procollagène est produit, ledit procédé étant autre qu'une méthode de traitement thérapeutique.

2. Procédé selon la revendication 1, dans lequel la séquence de reconnaissance est la séquence d'acides aminés GGQGSDPADV AIQL TFLRLM STE.

3. Procédé selon la revendication 1, dans lequel la séquence de reconnaissance est la séquence d'acides aminés NVEGVTSKEM ATQLAFMRLL ANY.

4. Procédé selon la revendication 1, dans lequel la séquence de reconnaissance est la séquence d'acides aminés GDDNLAPNTA NVQMTFLRLL STE.

5. Procédé selon la revendication 1, dans lequel la séquence de reconnaissance est la séquence d'acides aminés GNPELPEDVL DVQLAFLRLL SSR.

6. Procédé selon la revendication 1, dans lequel la séquence de reconnaissance est la séquence d'acides aminés VDAEGNPVGV VQMTFLRLL SAS.

7. Procédé selon la revendication 1, dans lequel la séquence de reconnaissance est la séquence d'acides aminés GDHQSPNTAI TQMTFLRLL SKE.

8. Procédé selon la revendication 1, dans lequel la séquence de reconnaissance est la séquence d'acides aminés LDVEGNSINM VQMTFLKLL TAS.

9. Procédé selon la revendication 1, dans lequel la séquence de reconnaissance est la séquence d'acides aminés VDSEGSPVGV VQLTFLRLL SVS.

10. Procédé selon l'une quelconque des revendications 1 à 9, dans lequel le gène manipulé d'une manière exogène code pour des chaînes pro-α formées de combinaisons de fragments de chaînes pro-α pro-α1 (I), pro-α2 (I), pro-α1 (II), pro-α1 (III), pro-α1 (V), pro-α2 (V), pro-α1 (XI) ou pro-α2 (XI).

11. Procédé selon la revendication 10, dans lequel le gène manipulé d'une manière exogène code une chaîne pro-α2 (I) modifiée dans laquelle la séquence de reconnaissance de la chaîne pro-α2 (I) a été substituée par la séquence de reconnaissance de la chaîne pro-α1 (III).

12. Procédé selon l'une quelconque des revendications précédentes, dans lequel le gène est incorporé dans un vecteur.

13. Procédé selon la revendication 12, dans lequel le vecteur est un plasmide, un cosmide ou un phage.

14. Procédé selon l'une quelconque des revendications précédentes, dans lequel le système est une cellule hôte transfectée avec le gène.

15. Procédé selon la revendication 14, dans lequel la cellule hôte est eucaryote.

16. Procédé selon la revendication 15, dans lequel la cellule hôte est une cellule de levure, d'insecte ou une cellule mammalienne.

17. Procédé selon la revendication 16, dans lequel la cellule hôte est une cellule mammalienne et est sélectionnée de fibroblastes ou de lignées cellulaires dérivées de ceux-ci, de cellules rénales de hamsters nouveau-nés, de cellules 3T3 de souris, de cellules ovariennes de hamster chinois ou de cellules COS.

18. Procédé selon l'une quelconque des revendications 1-14, dans lequel le système est une plante transgénique ou un animal non humain.

19. Procédé selon la revendication 18, dans lequel le système est un animal transgénique et est un mammifère placentaire non humain.

20. Procédé selon la revendication 19, dans lequel le mammifère placentaire est l'un quelconque d'entre du bétail, un mouton, une chèvre, un buffle d'eau, un chameau ou un cochon.
